# EUROPEAN PATENT SPECIFICATION

(11) **EP 3 419 264 B1**
(45) Date of publication and mention of the grant of the patent: **04.11.2020**
(21) Application number: 18178900.9
(22) Date of filing: 20.06.2018
(51) Int. Cl.: A44C 5/00, A61B 5/00, A61B 5/0205, H04M 1/725, H04B 1/3827, A44C 9/00

(54) **SENSORY INTERACTIVE SYSTEM FOR REMOTELY LOCATED ITEMS**
SENSORISCHES INTERAKTIVES SYSTEM FÜR FERNGEORTETE GEGENSTÄNDE
SYSTÈME INTERACTIF SENSORIEL POUR ARTICLES SITUÉS À DISTANCE

(30) Priority: 23.06.2017 IT 201700070607
(43) Date of publication of application: 26.12.2018
(73) Proprietor: Zemira Srls, 15121 Alessandria (IT)
(72) Inventor: PASSARIELLO, Teresa, 15121 Alessandria (IT); PASSARIELLO, Maria, 15121 Alessandria (IT)
(74) Representative: Maroscia, Antonio

(56) References cited:
- WO-A1-2015/114510
- WO-A1-2016/033575
- US-A1- 2016 150 362
- US-A1- 2017 180 921

## Description

### Field of The Invention

The present invention generally finds application in the field of accessories and manufacture of items having interactive electronic components, and particularly relates to an interactive sensory system for remotely located items.

### Background art

Electronic units have been long known to be employed in the field of items and devices designed to be worn by respective users, and to be integrated in the items and adapted to interact with each other.

Namely, interactive electronic units have been known to be widely used in the field of jewelry and the wearable items include jewels such as rings, bracelets, pendants and earrings.

These jewels generally comprise a rigid body containing a microprocessor control unit connected to a battery power source and connection means adapted to allow wireless connection between the jewel and a portable electronic device.

For example, TW201539249 discloses a ring comprising a body made of a metal material containing a control unit and connection means. The ring further comprises a memory unit and a microphone, the latter being selectively operable by a microswitch.

Particularly, using the connection means and in combination with the microphone and a gyroscope module, the ring can connect with a cell phone, a computer or other external electronic devices comprising an integrated operating system for data transmission and reception.

One drawback of this prior art arrangement is that the ring has very large dimensions and is very uncomfortable to wear.

Another drawback of this arrangement is that the electronic units in the jewel are not perfectly integrated therein and affect aesthetics.

A further drawback of this arrangement is that this jewel has a very complex construction and high manufacturing costs.

Yet another drawback is that users must manually actuate the control unit for data exchange to occur.

CN204166482 discloses a ring with a body made of a metal material associated with a control unit having a dedicated memory, a tactile sensor and a wireless connection means adapted for interaction with a portable electronic device.

Particularly, the control unit is integrated in the decoration of the ring, e.g. in a gem or a precious stone, for the aesthetics of the ring to be unaltered.

One drawback of this arrangement is that the jewel only acts as a peripheral device external to the portable electronic device and can interact with the latter only.

A further drawback is that the control unit and the connection means do not allow users to record or count the time spent together.

Another drawback is that the control unit does not allow data or information to be combined together and associated with a predetermined event.

Yet another drawback is that the sensors of the ring cannot sense the physical parameters of the user and associate them to the predetermined event.

US20170180921 discloses a sensor interactive system having all the features of the preamble of claim 1. WO2016033575 discloses a smart jewel comprising a microcontroller and a data memory connected to one or more connection bases adapted to accommodate a plurality of interchangeable expansions configured to communicate with the microcontroller and make the jewel a portable computer element configurable by a user. US20160150362 describes a bracelet for monitoring a user's position and health.

### Technical Problem

In the light of the prior art, the technical problem addressed by the present invention is to provide an interactive sensory system for remotely located items with respective integrated control units, adapted to afford direct connection therebetween for recording and combining digital data.

### Disclosure of the invention

The object of the present invention is to obviate the above drawback, by providing an interactive sensory system for remotely located items that is highly efficient and relatively cost-effective.

A particular object of the present invention is to provide a system as described above, in which the control units are configured to directly connect with each other.

A further object of the present invention is to provide a system as described above, in which the control units are configured to be automatically triggered as soon as a predetermined event is started.

A further object of the present invention is to provide a system as described above in which the control units allow detection and combination of digital data to associate it with a predetermined event.

A further object of the present invention is to provide a system as described above, in which the control units are integrated in the items and leave their aesthetics unaltered.

Another object of the present invention is to provide a system as described above that has a very small size.

A further object of the present invention is to provide a system as described above that is very simple to use.

Another object of the present invention is to provide a system as described above that can be simply manufactured and has very low costs.

A further object of the present invention is to provide a system as described above that affords constant monitoring of the physical functions of the users and warn about any changes thereto.

The aforementioned objects, as well as other objects that can be more clearly explained hereinafter, are fulfilled by an interactive sensory system for remotely located items according to claim 1 and comprising at least one pair of interactive items, at least one of which is adapted to be worn by a user, and each having a rigid body containing a microprocessor control unit having an internal clock and a computer program product having software code portions, wireless connection means connected to their respective control unit and a power source for supplying power to the microprocessor unit and connection means.

The connection means are configured to be activated when as the items are situated within a predetermined maximum distance and the clock operates with the connection means active and is configured to start counting the duration of the time intervals in which the items are within the predetermined maximum distance and to stop counting when the items of the pair are beyond the distance.

Advantageous embodiments of the invention are obtained in accordance with the dependent claims.

### Brief Description of The Drawings

Further features and advantages of the invention will be more apparent from the detailed description of a preferred, non-exclusive embodiment of an interactive sensory system of remotely located items according to the invention, which is described as a non-limiting example with the help of the following drawings, in which:
FIGS. 1 and 2 are schematic side views of a first embodiment of the interactive system in first and second operating configurations;
FIG. 3 is a schematic side view of a second embodiment of the interactive system;
FIG. 4 is a general block diagram of an embodiment of one of the items of the interactive system of the invention.

### Detailed description of a preferred exemplary embodiment

Particularly referring to the figures, there is shown an interactive sensory system for remotely-located items, generally designated by numeral 1.

The system 1 comprises at least one pair of interactive items 2, 3, at least one of which is designed to be worn by a user, and each having a rigid body 4.

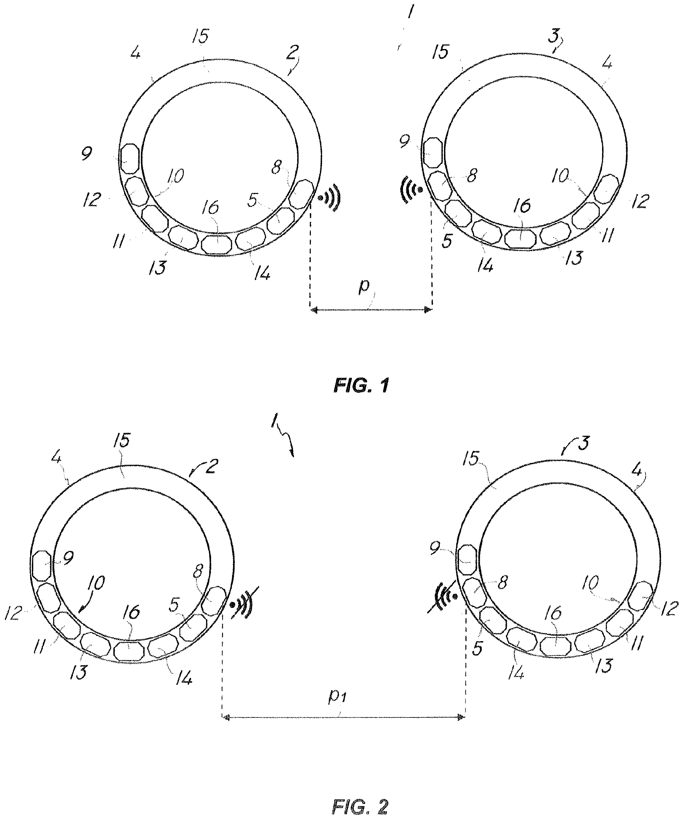

For example, in a first embodiment as shown in FIGS. 1 and 2, the items comprise a pair of jewels 2, 3, such as rings, bracelets, pendants or earrings, each adapted to be worn by a respective user and having a rigid body 4 made of a metal material or a jewelry material.

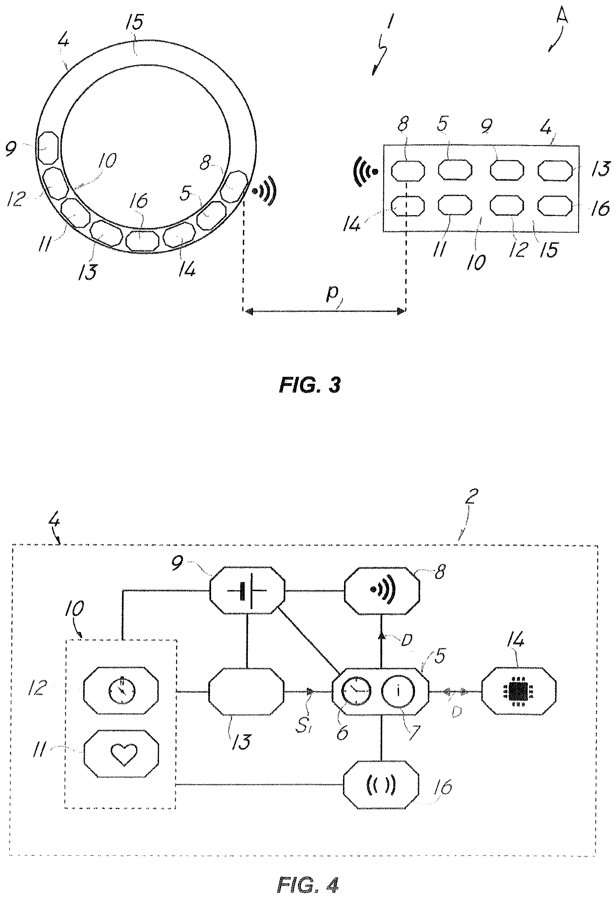

In an alternative embodiment of the invention, as shown in FIG. 3, the pair of items comprises a jewel 2 that is adapted to be worn by a user and a stationary base 3 which is located in a predetermined place A.

The rigid body 4 of the items 2, 3 comprises a respective microprocessor control unit 5 therein with an internal clock 6 and a computer program product 7 having software code portions.

In a preferred embodiment of the invention, each of the items 2, 3 comprises Bluetooth wireless connection means 8 which are configured to be triggered when the items 2, 3 are situated within a predetermined maximum distance p.

This distance p will depend on the type of the Bluetooth chip in use and then on the range thereof, and may range to 200 meters.

A power source 9 is provided, which may preferably comprise an induction charging battery for supplying power to the connection means 8 and the control unit 5.

This type of charging arrangement is known to comprise a first coil, located in a respective item and directly connected to the charging battery, having a current induced by a second coil in an external charger, not shown, flowing therethrough.

This external charger may be provided in the form of a header on which the item is placed during charging and having a cable with an end plug, adapted to be coupled to a home power source. Advantageously, the entire system 1 will have a low power consumption thereby enabling charging of the last for several consecutive days.

In a peculiar aspect of the invention, the clock 6 of the control unit 5 is operative when the connection means 8 are activated, then when the items 2, 3 are located within the predetermined distance p.

Upon enabling, the clock 6 is configured to count the duration of the time intervals in which the items 2, 3 are within the predetermined maximum distance p and to stop counting when the items 2, 3 are beyond the predetermined distance p, i.e. at a distance p₁ that is greater than the latter.

Conveniently, the durations of the time intervals as measured by the internal clock 6 will be processed and summed by the computer program product 7 installed in the control unit 5.

Therefore, in the first embodiment as described above, the two users wearing the two jewels 2, 3 may know the overall time that they spent together whereas, in an alternative embodiment, the user that wears the jewel 2 may know the overall time that he/she spent in a given place P, i.e. the place in which the stationary base 3 is located.

Both the jewels 2, 3 designed to be worn by users in the first embodiment, and the jewel 2 of the alternative embodiment comprise sensor means 10 in their respective rigid bodies 4, connected to their respective control units 5 and adapted to interact with the user for automatic sensing of its physical parameters and its position.

Namely, the sensor means 10 may comprise means 11 for detecting and monitoring the physical parameters of the users through tactile or optical sensors, not shown, that contact their skin.

These detection means 11 may detect physical condition data and vital signs of the user such as movement tracking, heartbeat rate, breathing rate, blood pressure and body temperature as well as their variation over time.

Likewise, the sensor means 10 may comprise geolocation means 12 accommodated in the respective rigid bodies 4 and adapted to interact with the outside for automatic sensing of the position of the item 2, 3 worn by the user, for example using a GPS chip and to locate the geographic location in which the clock 6 is actuated.

Advantageously, the jewels 2, 3 worn by the users may comprise one or more decorative elements, not shown, such as precious stones or gems, coupled to their respective rigid bodies 4.

The presence of these decorative elements does not alter the configuration and arrangement of the aforementioned parts or the connection between the two items 2, 3 in any manner.

In a preferred embodiment of the invention, the parameters detected by the above described sensor means 10 are transduced into electrical signals S_{I} through transducer means 13 located inside the jewels 2, 3 or the jewel 2 of the alternative embodiment and connected to their respective control unit 5.

The control unit 5 will be able to coordinate the execution of the tasks of the sensor means 10 and the transducer means 13 and to time them using the internal clock 6 of the system 1.

As the internal clock 6 is actuated, the geolocation sensor means 12 will be also actuated, whereas the detection means 11 may be activated before and after actuation of the clock 6, to measure the physical parameters at the two different times.

Furthermore, the computer program product 7 of the control unit 5 is configured to process the electrical signals S_{I} and convert them into digital data D indicative of the position and the physical parameters of the users to combine it with the durations of the time intervals as measured by the clock 6.

The digital data D and its combination with the measured time intervals, e.g. in association with a predetermined event in which both users are involved, may be stored in a memory unit 14 integrally incorporated in the body 4 of the items 2, 3 and directly connected to the control unit 5.

Thus, the users of the interactive items 2, 3 may access information concerning the time that they have spent within the predetermined maximum distance p and then the overall time that the users have spent together.

The memory unit 14 will have a small-size geometry, i.e. the form of a non-volatile solid-state flash memory, that can maintain the stored data even when it is not connected to the power source.

In a preferred embodiment of the invention, the connection means 8 will be configured to transmit and receive digital data D between the control units 5 of the items 2, 3 of the system 1.

In addition, the connection means 8 will be adapted to connect the control unit 5 with one or more portable electronic devices, not shown, including computer applications with respective interfaces to access the digital data transmitted by the control units 5 and stored in each memory unit 14.

These electronic devices may be selected from the group comprising smartphones, tablets, smartwatches and the like and the applications will be able to interact with the control unit 5 of each item 2, 3.

The applications may display through charts, numbers or images the total time spent by these items 2, 3 within the predetermined maximum distance p and the association of such time with a number of predetermined events in combination with the position and physical data D.

Furthermore, the computer application will allow the user to notify specific time intervals such as a given overall time period in which the clock 6 has been in operation.

In the first embodiment, the users of the items 2, 3 may access information concerning the places in which their respective clocks 6 were actuated by the applications, and hence the places in which both users of the system 1 were.

Furthermore, the application will concatenate information of the pictures taken by the portable electronic device associated with one of the items 2 during actuation of the clock 6 and share such information with the electronic device associated with the other item 3.

In a similar manner, the application may access the data D stored by the item 2, 3 during actuation of the clock 6 to assess the process and variations of the physical parameters of the user.

In an alternative embodiment, not shown, the system 1 may be configured by these applications for remote or emergency monitoring of the exact position of one of the items 2, 3 worn by a user, for example in cases of involuntary departure of the user, even when even when the clock 6 is off.

Likewise, the system 1 may have a feature that affords real-time monitoring of the vital functions of the user for storage and later analysis of particular diseases.

As best shown in FIGS. 1 and 2, the sensor means 10, the transducer means 13, the control unit 5 and the connection means 8 are housed in the body 4 of their respective items 2, 3, preferably in a cavity 15 formed during jewel processing, such that the aesthetics and the overall dimensions of the jewel will remain unaltered.

Advantageously, all of the above listed parts may be mounted to a box-like grid of a predetermined size, not shown, which is placed in the cavity 15.

The system 1 may include alarm means 16 connected to the control unit 5, the connecting means 8 and the sensor means 10 to warn the user at the time of actuation of the clock 6 or variation of of his/her physical parameters and position.

These alarm means 16 may be embodied by a vibration chip that can inform the user about a particular event occurring after actuation of the clock 6.

For example, in a system 1 composed of a pair of items 2, 3 of the invention, each worn by a user, the vibration chip 16 may inform the first user about a heart rate change of the second user when the pair of items 2, 3 interact within the predetermined distance p.

Furthermore, the alarm means 16 and the connection means 8 may interact with computer applications to allow the portable electronic device to send notifications in case of alteration of the physical parameters even when the interactive items 2, 3 are located beyond the predetermined distance p.

It will be appreciated from the foregoing that the sensory interactive jewel system of the invention fulfills the intended objects and particularly affords recording, processing and combining of user data, while not altering the appearance of the jewels.

While the system has been described with particular reference to the accompanying figures, the numerals referred to in the disclosure and claims are only used for the sake of a better intelligibility of the invention and shall not be intended to limit the claimed scope in any manner.

### Industrial Applicability

The present invention may find application in industry, because it can be produced on an industrial scale in the accessories industry, particularly in goldsmith's craft and jewelry.

## Claims

1. A sensory interactive system (1) for remotely located items, comprising:
- at least one pair of interactive items (2, 3), at least one of which is adapted to be worn by a user, wherein each of said items (2, 3) has a rigid body (4) accommodating internally thereof;
- a microprocessor control unit (5) having an internal clock (6) and a computer program product (7) with software code portions;
- wireless connection means (8) connected to their respective microprocessor control unit (5);
- a power source (9) for supplying power to said microprocessor control unit (5) and said connection means (8);
wherein said connection means (8) are configured to be activated when the items (2, 3) are within a predetermined maximum distance (p) and
wherein said clock (6) is operative when said connection means (8) are activated;
**characterized in that** said clock (6) is configured to start counting the duration of the time periods in which the items (2, 3) of said at least one pair are within said predetermined maximum distance (p) and to stop counting when the items (2, 3) of said pair are beyond said distance (p), said computer product (7) being adapted to process and sum the lengths of the time periods as measured by said clock (6), said at least one wearable item (2) comprising sensor means (10) which are adapted to interact with the user and the outside environment for automatically detecting physical and position parameters, as well as transducer means (13) for transducing the parameters detected by said sensor means (10) into electrical signals (S_{I}), said sensor means (10) and said transducer means (13) being connected to their respective microprocessor control unit (5) and said computer program product (7) being configured to process said electrical signals (S_{I}) into digital data (D), said sensor means (10) comprising means (11) for detecting and monitoring physical parameters of the users before and after actuation of the respective clock (6).

2. System as claimed in claim 1, whereby said items (2, 3) comprise a pair of jewels, each adapted to be worn by a respective user, said wearable jewels (2, 3) comprising respective said sensor means (10) and said transducer means (13).

3. System as claimed in claim 1, whereby the items (2, 3) of said pair comprise a jewel (2) which is adapted to be worn by a user and has respective sensor means (10) and transducer means (13) and a stationary base (3) located in a predetermined place (P), which has no sensor or transducer means and is equipped with said connection means (8).

4. System as claimed in claim 1, whereby said connection means (8) are of Bluetooth type and are configured to transmit and receive said digital data (D) between the microprocessor control units (5) of the items (2, 3) of each pair.

5. System as claimed in claim 1, whereby said connection means (8) are adapted to connect said microprocessor control units (5) with one or more portable electronic devices having applications and respective interfaces loaded thereon for accessing said digital data (D) transmitted by said control units (5).

6. System as claimed in claim 1, whereby said sensor means (10) comprise geolocation means (12) which are configured to find the geographic location in which the clock (6) is actuated.

7. System as claimed in claim 1, whereby said power source (9) comprises an induction charging battery.

8. System as claimed in claim 1, whereby it comprises alarm means (16) connected to said microprocessor control unit (5), said sensor means (10) and said connection means (8) to inform the user upon actuation of said clock (6) or variation of said position or physical parameters.

## Patentansprüche

1. Ein sensorisch interaktives System (1) für entfernt lokalisierte Gegenstände, umfassend:
- mindestens ein Paar interaktiver Gegenstände (2, 3), von denen mindestens einer zum Tragen durch einen Benutzer geeignet ist, wobei jeder der Gegenstände (2, 3) einen starren Körper (4) aufweist, der innen darin untergebracht ist;
- eine Mikroprozessorsteuereinheit (5) mit einer internen Uhr (6) und einem Computerprogrammprodukt (7) mit Softwarecodeabschnitten;
- drahtlose Verbindungmittel (8), die mit der jeweiligen Mikroprozessorsteuereinheit (5) verbunden sind;
- eine Stromquelle (9) zum Versorgen mit Strom der Mikroprozessorsteuereinheit (5) und den Verbindungsmittel (8);
wobei die Verbindungsmittel (8) so konfiguriert sind, dass sie aktiviert werden, wenn sich die Gegenstände (2, 3) innerhalb eines vorbestimmten maximalen Abstands (p) befinden, und
wobei die Uhr (6) wirksam ist, wenn die Verbindungsmittel (8) aktiviert sind;
**dadurch gekennzeichnet, dass** die Uhr (6) so konfiguriert ist, dass sie mit dem Zählen der Dauer der Zeiträume beginnt, in denen sich die Gegenstände (2, 3) des mindestens einen Paares innerhalb des vorbestimmten maximalen Abstands (p) befinden, und mit dem Zählen aufhört, wenn die Gegenstände (2, 3) des Paares jenseits des Abstands (p) sind, wobei das Computerprodukt (7) angepasst ist, um die durch die Uhr (6) gemessenen Längen der Zeiträume zu verarbeiten und zu summieren, wobei indestens ein tragbarer Gegenstand (2) Sensormittel (10) umfasst, die angepasst sind, um mit dem Benutzer und der Außenumgebung zur automatischen Erfassung von physikalischen Parametern und Positionsparametern zu interagieren, und ausserdem Wandlermittel (13) zum Umwandeln der von den Sensormitteln (10) erfassten Parameter in elektrische Signale (S_{I}) umfasst, wobei die Sensormittel (10) und die Wandlermittel (13) mit ihrer jeweiligen Mikroprozessorsteuereinheit (5) verbunden sind und das Computerprogrammprodukt (7) konfiguriert ist, um die elektrischen Signale (S_{I}) in digitale Daten (D), wobei die Sensormittel (10) eine Einrichtung (11) zum Erfassen und Überwachen physikalischer Parameter der Benutzer vor und nach dem Betätigen der jeweiligen Uhr (6) umfasst.

2. System nach Anspruch 1, wobei die Gegenstände (2, 3) ein Paar Juwelen umfassen, die jeweils zum Tragen durch einen jeweiligen Benutzer angepasst sind, wobei die tragbaren Juwelen (2, 3) jeweils die Sensormittel (10) und die Wandlermittel (13) umfassen.

3. System nach Anspruch 1, wobei die Gegenstände (2, 3) des Paares ein Juwel (2) umfassen, das zum Tragen durch einen Benutzer geeignet ist und entsprechende Sensormittel (10) und Wandlermittel (13) und eine stationäre Basis (3) aufweist, die an einer vorbestimmten Stelle (P) angeordnet ist, die kein Sensor- oder Wandlermittel aufweist und mit diesen Verbindungsmitteln (8) ausgestattet ist.

4. System nach Anspruch 1, wobei die Verbindungsmittel (8) vom Bluetooth-Typ sind und konfiguriert sind, um die digitalen Daten (D) zwischen den Mikroprozessor-Steuereinheiten (5) der Gegenstände (2, 3) jedes Paares zu senden und zu empfangen.

5. System nach Anspruch 1, wobei die Verbindungsmittel (8) angepasst sind, um die Mikroprozessorsteuereinheiten (5) mit einem oder mehreren tragbaren elektronischen Geräten zu verbinden, wobei Anwendungen und entsprechende Schnittstellen zum Zugreifen auf die von den Steuereinheiten (5) übertragene digitale Daten (D) darauf geladen sind.

6. System nach Anspruch 1, wobei die Sensoreinrichtung (10) Geolokalisierungsmittel (12) umfasst, die konfiguriert sind, um den geografischen Ort zu finden, an dem die Uhr (6) betätigt wird.

7. System nach Anspruch 1, wobei die Stromquelle (9) eine Induktionsladebatterie umfasst.

8. System nach Anspruch 1, wobei es Alarmmittel (16) umfasst, die mit der Mikroprozessorsteuereinheit (5), den Sensormitteln (10) und den Verbindungsmitteln (8) verbunden sind, um den Benutzer bei Betätigung der Uhr (6) oder die Änderung der Position oder der physikalischen Parameter zu informieren.

## Revendications

1. Un système d'interaction sensorielle (1) pour des articles situés à distance, comprenant:
- au moins une paire d'articles interactifs (2, 3), dont au moins l'un est adapté pour être porté par un utilisateur, dans lequel chacun desdits articles (2, 3) a un corps rigide (4) logeant à l'intérieur de celui-ci;
- une unité de commande à microprocesseur (5) ayant une horloge interne (6) et un produit programme informatique (7) avec des parties de code logiciel;
- un moyen de connexion sans fil (8) connecté à l'unité de commande de microprocesseur respective (5);
- une source d'alimentation (9) pour fournir de l'énergie à ladite unité de commande à microprocesseur (5) et auxdits moyens de connexion (8);
dans lequel lesdits moyens de connexion (8) sont configurés pour être activés lorsque les éléments (2, 3) sont à l'intérieur d'une distance maximale prédéterminée (p) et
dans lequel ladite horloge (6) est opérationnelle lorsque lesdits moyens de connexion (8) sont activés;
**caractérisé en ce que** ladite horloge (6) est configurée pour commencer à compter la durée des périodes de temps pendant lesquelles les articles (2, 3) de ladite au moins une paire sont à l'intérieur de ladite distance maximale prédéterminée (p) et pour arrêter le comptage lorsque les articles (2, 3) de ladite paire sont au-delà de ladite distance (p), ledit produit informatique (7) étant adapté pour traiter et additionner les longueurs des périodes de temps telles que mesurées par ladite horloge (6), ledit au moins un article portable (2) comprenant des moyens capteurs (10) qui sont adaptés pour interagir avec l'utilisateur et l'environnement extérieur pour détecter automatiquement les paramètres physiques et de position, ainsi que des moyens transducteurs (13) pour transduire les paramètres détectés par lesdits moyens capteurs (10) en signaux électriques (Si), lesdits moyens capteurs (10) et lesdits moyens transducteurs (13) étant connectés à leur respective unité de commande du microprocesseur (5) et ledit produit de programme informatique (7) étant configuré pour traiter lesdits signaux électriques (Si) en données numériques (D), lesdits moyens capteurs (10) comprenant des moyens (11) pour détecter et surveiller les paramètres physiques des utilisateurs avant et après l'actionnement de l'horloge respective (6).

2. Système selon la revendication 1, dans lequel lesdits articles (2, 3) comprennent une paire de bijoux, chacun étant adapté pour être porté par un utilisateur respectif, lesdits bijoux portables (2, 3) comprenant respectivement lesdits moyens de détection (10) et lesdits moyens transducteurs (13).

3. Système selon la revendication 1, dans lequel les articles (2, 3) de ladite paire comprennent un bijou (2) qui est adapté pour être porté par un utilisateur et a respectifs moyens capteurs (10) et moyens transducteurs (13) et une base fixe (3) située à un emplacement prédéterminé (P), qui n'a ni moyens capteurs ni transducteurs et est équipée desdits moyens de connexion (8).

4. Système selon la revendication 1, dans lequel lesdits moyens de connexion (8) sont de type Bluetooth et sont configurés pour transmettre et recevoir lesdites données numériques (D) entre les unités de commande du microprocesseur (5) des articles (2, 3) de chacun paire.

5. Système selon la revendication 1, dans lequel lesdits moyens de connexion (8) sont adaptés pour connecter lesdites unités de commande du microprocesseur (5) à un ou plusieurs dispositifs électroniques portables ayant des applications et des interfaces respectives chargées dessus pour accéder auxdites données numériques (D) transmises par lesdites unités de commande (5).

6. Système selon la revendication 1, dans lequel lesdits moyens capteurs (10) comprennent des moyens de géolocalisation (12) qui sont configurés pour trouver l'emplacement géographique dans lequel l'horloge (6) est actionnée.

7. Système selon la revendication 1, dans lequel ladite source d'énergie (9) comprend une batterie de chargement par induction.

8. Système selon la revendication 1 dans lequel il comprend des moyens d'alarme (16) connectés à ladite unité de commande du microprocesseur (5), auxdits moyens capteurs (10) et auxdits moyens de connexion (8) pour informer l'utilisateur lors de l'actionnement de ladite horloge (6) ou d'une variation de ladite position ou des paramètres physiques.
